# EUROPEAN PATENT APPLICATION

(11) **EP 4 645 330 A1**
(43) Date of publication of application: **05.11.2025**
(21) Application number: 24840155.6
(22) Date of filing: 24.06.2024
(51) Int. Cl.: G16H 20/70, G16H 10/20, G16H 10/60, G16H 50/30, G16H 50/20, A61B 5/16, A61B 5/00

(54) **DIGITAL THERAPY SYSTEM BASED ON COGNITIVE BEHAVIORAL THERAPY AND METHOD THEREOF**

(30) Priority: 11.07.2023 KR 20230089685
(71) Applicant: Neudive Inc., Daegu 41061 (KR)
(72) Inventor: CHO, Sung Ja, Seoul 08727 (KR)
(74) Representative: Krauns, Christian
(86) International application number: PCT/KR2024/095887
(87) International publication number: WO 2025/014345

(57) **Abstract**

A Cognitive Behavioral Therapy-based digital therapeutic system according to some embodiments of the present invention includes a user terminal and a digital treatment server that provides Cognitive Behavioral Therapy-based learning content to the user terminal. The digital treatment server includes an evaluation unit that evaluates the user's learning result based on the user's response information received from the user terminal. The evaluation unit includes a validity determination unit that evaluates the validity of the response information and a learning evaluation unit that evaluates the user's learning based on the response information.

## Description

### TECHNICAL FIELD

The present invention relates to a digital therapeutic system based on cognitive behavioral therapy and a method thereof, and more particularly, to a digital therapeutic system based on cognitive behavioral therapy and a method thereof that can improve the reliability of learning result evaluation by first determining the validity of user response information when evaluating the learning results of a user with autism spectrum disorder.

### BACKGROUND OF THE INVENTION

Autism spectrum disorder is a mental illness characterized by developmental defects in social interaction, limited interests, stereotyped behaviors, and sensory sensitivities, appearing from early life and lasting throughout life. Individuals with autism spectrum disorder exhibit developmental delays in various aspects such as cognition, language, social-emotional, physical, and behavior from infancy. Developmental delays due to autism spectrum disorder affect various areas of life such as school life, occupation, social relationships, daily life, and independence, and in most cases, require the help of those around them, such as family, teachers, and activity assistants, due to difficulties in social interaction and safety issues. Therefore, discovering and treating autism spectrum disorder has a significant impact on a child's long-term growth and development, and can have a positive impact on families and society as a whole.

However, the development of effective treatments for social interaction deficits in individuals with autism spectrum disorder is slow, and utilization is low due to high costs and limited treatment facilities. While risperidone and aripiprazole have received FDA approval for treating irritability associated with autism spectrum disorder, there are no FDA-approved standard drugs that target core symptoms such as social situation awareness and interaction problems. According to the American Association of People with Disabilities, the average treatment expenditure for children with autism spectrum disorder in the United States in 2017 was $17,081 per year. Furthermore, including not only direct treatment expenses incurred by parents of children with autism spectrum disorder but also indirect costs (e.g., loss of parents' working hours, household labor costs), the average household treatment expenditure is estimated to be over $36,000 per year. In Korea, Applied Behavior Analysis (ABA) has been introduced for the promotion of social interaction in autism spectrum disorder for over 10 years, and treatment programs are gradually becoming widespread. However, in Korea, the number of certified therapists is small and concentrated in the metropolitan area, and the utilization rate is low due to costs of nearly 60,000 to 100,000 won per session, leading to monthly medical expenses exceeding 3,000,000 won. Additionally, when utilizing services such as rehabilitation exercise therapy, speech therapy, art therapy, and music therapy, additional costs may be incurred, and 49.7% of parents of children with developmental disabilities responded that treatment costs are 'very burdensome' or 'somewhat burdensome'.

Meanwhile, digital therapeutic devices help children learn in an engaging way, providing content in a manner that children can easily understand and find interesting by utilizing games, videos, and animations. This approach can help children view learning as fun, build confidence, and participate actively. Digital therapeutic devices are portable as they can be applied using smartphones, smartpads, etc., are not restricted by location, allow for multiple repetitions of learning, and are relatively inexpensive compared to face-to-face therapy. In particular, they are considered useful for solving daily life problems by exchanging feedback in real time when they occur.

In particular, individuals with autism spectrum disorder exhibit qualitative problems in social interaction, impairments in language and communication, and sometimes instability in mood and emotion. Furthermore, they use certain language in a stereotyped or repetitive manner.

Regarding social interaction, individuals with autism spectrum disorder lack cognitive empathy, experiencing difficulty in reading and understanding others' emotions, and simultaneously struggling to recognize how to connect their own emotions with cognitive expressions. For autism spectrum disorder, speech therapy is essential to improve communication skills. Cognitive empathy in autism spectrum disorder is acquired through learning and is not automatically internalized unlike in neurotypical individuals.

Furthermore, regarding communication, individuals with autism spectrum disorder exhibit difficulties in communication due to limited, inappropriate, or unique language use. They may also have monotonous speech pitch, unique intonation or rhythm, or excessively high or low vocal tone. When speaking, individuals with autism spectrum disorder may exhibit abnormalities such as inaccurate pronunciation, incoherence, or a mix of informal and formal language. Individuals with autism spectrum disorder find it even more difficult to converse with others. For example, an individual with autism spectrum disorder might inappropriately mimic the angry tone of a serious speaker in a mocking manner, laughing and giggling.

Therefore, to solve the aforementioned problems, a training program is needed for content such as emotion recognition and differentiation using digital therapeutic devices, specific methods used in social situations, problem-solving skills in interpersonal relationships, and social communication. Especially in speech therapy using digital therapeutic devices, since it is essential to evaluate treatment results based on the responses of individuals with autism spectrum disorder, the necessity for a digital therapeutic system and method for individuals with autism spectrum disorder using digital therapeutic devices has emerged, which can first determine the validity of the responses and then evaluate learning results only when the responses are valid.

### BRIEF SUMMARY OF THE INVENTION

### PROBLEM TO BE SOLVED

An object of the present invention to solve the aforementioned problems is to provide a digital therapeutic system based on cognitive behavioral therapy and a method thereof that can receive responses from individuals with autism spectrum disorder who have received learning and therapy using a digital therapeutic device, and determine the validity of the received responses.

Another object of the present invention to solve the aforementioned problems is to provide a digital therapeutic system based on cognitive behavioral therapy and a method thereof that can enhance daily life-oriented executive functions of individuals with autism spectrum disorder using a digital therapeutic device.

### SOLUTION TO PROBLEM

To achieve the aforementioned objects, a digital therapeutic system based on cognitive behavioral therapy according to an embodiment of the present invention includes a user terminal; and a digital therapeutic server configured to provide learning content based on cognitive behavioral therapy to the user terminal, wherein the digital therapeutic server includes an evaluation unit configured to evaluate a user's learning result based on user response information received from the user terminal, and wherein the evaluation unit may include a validity determination unit configured to evaluate the validity of the response information and a learning evaluation unit configured to evaluate the user's learning result based on the response information.

In this case, the response information may be selection option information or utterance information received from the user terminal.

According to some embodiments, the validity determination unit may evaluate the validity of the response information based on a conversation response speed evaluation criterion that evaluates whether the user responded at an appropriate time after an interlocutor's speech ended.

According to some embodiments, when evaluating the validity of the response information based on the conversation response speed evaluation criterion, the validity determination unit may determine that the user responded at a time when echolalia included in the response information ended, in a case where the response information includes the echolalia.

According to some embodiments, when evaluating the validity of the response information, the validity determination unit may extract emotion from the user's utterance by analyzing the response information, and evaluate the validity of the response information by comparing the extracted emotion with an emotion included in model response information.

According to some embodiments, when evaluating the user's learning, the learning evaluation unit may evaluate a similarity between the user's response information and a model response.

According to some embodiments, in a case where the response information includes a mispronounced word, the learning evaluation unit may replace the mispronounced word with a correctly pronounced word to evaluate the user's learning.

According to some embodiments, the digital therapeutic server further includes a pronunciation unit configured to receive pronunciation information from the user, wherein the pronunciation unit is configured to transmit first data including text information to be uttered by the user to the user terminal, and wherein the user terminal is configured to generate second data including pronunciation information generated by having the user pronounce the text information included in the received first data, and transmit the second data to the pronunciation unit.

According to some embodiments, the pronunciation unit is configured to analyze the pronunciation information to extract a mispronounced word of the user, and store the mispronounced word by matching it with a correctly pronounced word corresponding to the mispronounced word, and wherein the learning evaluation unit is configured to evaluate the user's learning by replacing the mispronounced word included in the response information with the correctly pronounced word based on the mispronounced word extracted from the pronunciation information.

According to some embodiments, the second data further includes user meta information, wherein the meta information includes user's age, gender, and disability level information.

According to some embodiments, when evaluating the user's learning, the learning evaluation unit may evaluate a similarity between the user's response information and the pronunciation information.

A digital therapeutic method based on cognitive behavioral therapy according to some embodiments of the present invention includes a step of evaluating user response information received from a user terminal, wherein the step of evaluating the response information includes a step of evaluating the validity of the response information and a step of evaluating the user's learning based on the response information, and wherein the step of evaluating the validity may evaluate the validity of the response information based on a conversation response speed evaluation criterion that evaluates whether the user responded at an appropriate time after an interlocutor's speech ended.

### ADVANTAGES OF THE INVENTION

According to some embodiments of the digital therapeutic system based on cognitive behavioral therapy and method thereof, it is possible to determine the validity of response information received from an individual with autism spectrum disorder before evaluating the learning results based on that response information, thereby improving the reliability of learning result evaluation.

Furthermore, by providing educational content based on cognitive behavioral therapy to individuals with autism spectrum disorder, it has the effect of achieving outcomes such as cognitive improvement, behavior modification, and emotion regulation through repetitive learning.

Furthermore, by providing educational content based on cognitive behavioral therapy to individuals with autism spectrum disorder, it has the effect of achieving outcomes such as cognitive improvement, behavior modification, and emotion regulation through repetitive learning.

Moreover, by providing educational content to individuals with autism spectrum disorder through a digital therapeutic device, it offers advantages such as good portability, no location restrictions, capability for multiple repetitive learnings, and being relatively inexpensive compared to face-to-face therapy.

Furthermore, by providing educational content to individuals with autism spectrum disorder using games, videos, and animations, it has the effect of allowing individuals with autism spectrum disorder to understand the educational content more easily and feel more interested.

### BRIEF DESCRIPTION OF DRAWINGS

FIG. 1 is a block diagram showing each configuration of a digital therapeutic system based on cognitive behavioral therapy according to the present invention.
FIG. 2 is a block diagram showing each configuration of a digital therapeutic server according to the present invention.
FIG. 3 is a diagram illustrating an example of topics presented by a presentation unit to a user terminal.
FIG. 4 is a flowchart of a digital therapeutic method based on cognitive behavioral therapy according to the present invention.

### DETAILED DESCRIPTION OF EMBODIMENTS

Hereinafter, some embodiments of the present invention will be described in detail with reference to exemplary drawings. In adding reference numerals to the constituent elements of each drawing, it should be noted that the same reference numerals are used as much as possible even if they are displayed on different drawings. In describing embodiments of the present invention, detailed descriptions of related known configurations or functions will be omitted if it is determined that they hinder understanding of the embodiments of the present invention.

In describing constituent elements of embodiments of the present invention, terms such as first, second, A, B, (a), and (b) may be used. Such terms are only for distinguishing the constituent element from other constituent elements, and the nature, order, or sequence of the corresponding constituent element is not limited by the terms. In this specification, the singular form also includes the plural form unless specifically mentioned in the phrase. As used herein, the terms "comprises" and/or "comprising" do not exclude the presence or addition of one or more other constituent elements besides the mentioned constituent elements.

Hereinafter, the present invention will be described in more detail with reference to the accompanying drawings.

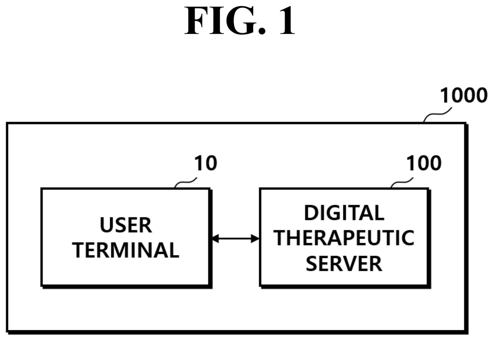

FIG. 1 is a block diagram showing each configuration of a digital therapeutic system 1000 based on cognitive behavioral therapy according to the present invention.

A digital therapeutic system 1000 based on cognitive behavioral therapy according to the present invention (hereinafter, referred to as 'system') includes a user terminal 10 and a digital therapeutic server 100.

The user terminal 10 (digital therapeutic device, hereinafter, referred to as 'user terminal 10') may be a terminal carried by a user with autism spectrum disorder. A user can transmit and receive information with the digital therapeutic server 100 through the user terminal 10. An application for treating the user's autism spectrum disorder may be installed in the user terminal 10. The application is a user program for specifically performing the system and method according to the present invention. Such an application may be downloaded and installed from the digital therapeutic server 100 or another app store server. The application installed in the user terminal 10 can communicate with the digital therapeutic server 100 to receive learning content, display learning screens within the learning content on the display device of the user terminal 10, utter audio information, or record the user's utterance.

The digital therapeutic server 100 can provide learning content based on cognitive behavioral therapy to the user terminal 10. Meanwhile, in this specification, the learning content includes basic content provided by a learning unit 130, skill reinforcement content provided by an application unit 140, and virtual field content provided by a virtual field unit 150, which will be described later. The digital therapeutic server 100 provides at least one communication protocol for communicating with the user terminal 10 through a communication network, and may provide an interface such as an Application Programming Interface (API) for app interworking to the user terminal 10 for service regarding the application.

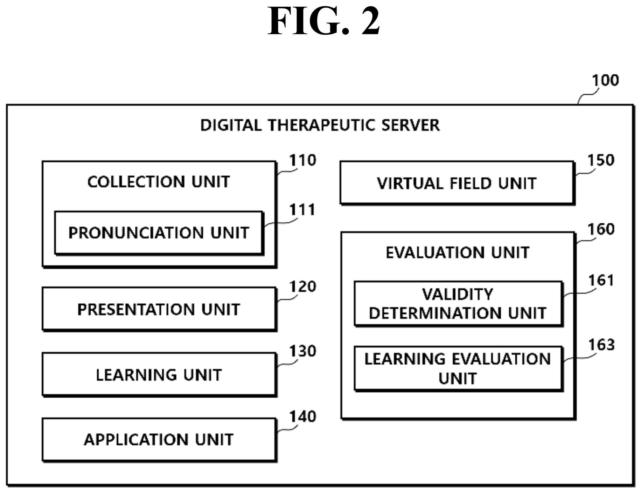

Hereinafter, each configuration of the digital therapeutic server 100 according to the present invention will be described in detail with reference to FIG. 2.

FIG. 2 is a block diagram showing each configuration of the digital therapeutic server 100 according to the present invention.

The digital therapeutic server 100 according to the present invention, referring to FIG. 2, includes a collection unit 110, a presentation unit 120, a learning unit 130, an application unit 140, a virtual field unit 150, and an evaluation unit 160.

Meanwhile, adolescents and adults with autism spectrum disorder experience serious problems with daily life adaptation functions due to executive function problems, even in the absence of intellectual disability. In particular, they often have difficulty completing goal-directed behavior due to problems with key executive functions such as cognitive flexibility, self-control of emotions and impulses, working memory, and planning and initiation.

Studies have been published that identify individual executive function vulnerabilities in people with autism spectrum disorder and demonstrate the effectiveness of providing customized programs for strengthening executive functions. These studies have shown improvements in working memory, behavioral control, and planning and organizational skills among executive functions, and also proven effective in reducing anxiety. However, these studies were mostly conducted under controlled experimental conditions and reported only limited indirect effects in the process of increasing adaptability and social interaction in daily life, thus having limitations in claiming to have performed real-world based social behavior promotion.

Therefore, the system according to the present invention includes: ① a collection unit 110 identifies the user's current knowledge (background information) through the user terminal 10; ② a presentation unit 120 presents at least one topic to the user terminal 10 and receives a selection of one of the at least one topic from the user terminal 10; ③ a learning unit 130, when the user terminal 10 selects one of the at least one topic, provides basic content that can be repeatedly practiced along with a plurality of detailed tasks according to that topic to the user terminal 10 to enable skill acquisition; ④ an application unit 140 provides skill reinforcement content to the user terminal 10 to enable the application of skills acquired through the basic content; ⑤ a virtual field unit 150 provides virtual field content that reconstructs the content of tasks or skill reinforcement content derivable from the basic content to promote repetitive practice in a constantly changing environment; and ⑥ an evaluation unit 160 evaluates the user's learning results to educate and treat individuals with autism spectrum disorder in terms of adaptability in daily life and social interaction.

In particular, the evaluation unit 160 according to the present invention can evaluate the user's learning results based on the user's response information received from the user terminal 10. Meanwhile, the user's response information, which is the subject of evaluation by the evaluation unit 160, may include option information and/or utterance information received from the user terminal 10, and a brief description thereof is as follows.

The learning unit 130, the application unit 140, and the virtual field unit 150 according to the present invention can each provide basic content, skill reinforcement content, and virtual field content to the user terminal 10.

As described above, when the user terminal 10 selects one of at least one topic, the learning unit 130 can provide basic content that can be repeatedly practiced along with a plurality of detailed tasks according to the selected topic to the user terminal 10. In this case, the basic content consists of videos, each including scenes corresponding to tasks according to the topic selected by the user. The basic content may, for example, consist of an animation in which multiple speakers converse with each other. The video of the basic content may include not only the conversations of multiple speakers but also separate configurations for explaining each topic and task and for helping the user understand the current situation.

The application unit 140, as described above, can provide skill reinforcement content to the user terminal 10 that allows the application of skills acquired through the basic content. In this case, the skill reinforcement content is centered on a method in which multiple speakers converse with each other to allow concentration solely on conversation training based on the conversation content included in the basic content. Here, one of the speakers performs the role of the user, and the skill reinforcement content includes first skill reinforcement content and second skill reinforcement content in which only the utterances of the speaker performing the role of the user in the first skill reinforcement content are muted. The user can repeatedly learn conversation training through the first skill reinforcement content. After repeatedly learning conversation training through the first skill reinforcement content, the user can learn verbal conversation expressions by completing the omitted conversation when the muted speaker utters through the second skill reinforcement content.

In this case, when the conversation of the character representing the user is omitted in the second skill reinforcement content, the application unit 140 can display a plurality of options including the appropriate utterance content of that character (i.e., an exemplary response corresponding to the utterance of the speaker repeatedly learned in the first skill reinforcement content) on the user terminal 10. The user can select an option that contains the appropriate utterance from the displayed options. Alternatively, when the conversation of the character representing the user is omitted in the second skill reinforcement content, the application unit 140 can provide an environment where the user can actually utter the appropriate utterance content of that character. When the muted speaker's utterance is provided during the playback of the second skill reinforcement content, the user terminal 10 can utter an exemplary response corresponding to the utterance of the speaker repeatedly learned in the first skill reinforcement content, and the user terminal 10 can record the user's utterance. The user terminal 10 transmits the user's selected option information and/or recorded utterance information to the application unit 140, and the application unit 140 can transmit the received option information and/or recorded utterance information to the evaluation unit 160. The evaluation unit 160 can evaluate the user's learning results based on the received option information and/or utterance information.

Furthermore, the virtual field unit 150, as described above, can provide virtual field content to the user terminal 10, which reconstructs the content of tasks or skill reinforcement content that can be derived from topics and/or basic content. The virtual field content is intended for learning and educating how to appropriately cope with various changing situations, considering the characteristics of individuals with autism spectrum disorder who find it difficult to flexibly adapt and apply according to various subsequent changes. The virtual field unit 150 provides options to allow the user to select appropriate utterances in a virtual field related to the topic presented by the presentation unit 120, or provides an environment where the user can actually utter, thereby allowing the user to apply the acquired skills in a virtual field.

The virtual field content, like the aforementioned skill reinforcement content, includes a speaker performing the user's role, and may include first virtual field content with the full content of the utterance, and second virtual field content where the utterance of the speaker performing the user's role is muted. The user can repeatedly learn conversation training that may occur in a virtual field assuming various changing situations through the first virtual field content. After repeatedly learning conversation training through the first virtual field content, the user can learn verbal conversation expressions by completing the omitted conversation when the muted speaker utters through the second virtual field content.

In this case, when the conversation of the character representing the user is omitted in the second virtual field content, the virtual field unit 150 can display a plurality of options including the appropriate utterance content of that character (i.e., an exemplary response corresponding to the utterance of the speaker repeatedly learned in the first virtual field content) on the user terminal 10. The user can select an option that contains the appropriate utterance from the displayed options. Alternatively, when the conversation of the character representing the user is omitted in the second virtual field content, the virtual field unit 150 can provide an environment where the user can actually utter the appropriate utterance content of that character. When the muted speaker's utterance is provided during the playback of the second virtual field content, the user terminal 10 utters an exemplary response corresponding to the utterance of the speaker repeatedly learned in the first virtual field content, and the user terminal 10 records the user's utterance. The user terminal 10 transmits the user's selected option information and/or recorded utterance information to the virtual field unit 150, and the virtual field unit 150 can transmit the received option information and/or recorded utterance information to the evaluation unit 160. The evaluation unit 160 can evaluate the user's learning results based on the received option information and/or utterance information.

The evaluation unit 160 receives user's response information from the application unit 140 and the virtual field unit 150, respectively, and can evaluate the user's learning results learned in the skill reinforcement content and the virtual field content, respectively, based on the received response information. Meanwhile, the digital therapeutic system 1000 according to the present invention can evaluate the user's response information by textifying utterance information through presented option information via text information or through a known method to generate text information, and then comparing the generated text information with an exemplary response that also includes text information.

The evaluation unit 160 includes a validity determination unit 161 and a learning evaluation unit 163. The validity determination unit 161 can evaluate the validity of the user's response information received from the user terminal 10. The learning evaluation unit 163 can evaluate the user's learning results based on that response information. When the evaluation unit 160 displays the user's learning results evaluated by the learning evaluation unit 163 on the user terminal 10, a guardian terminal, or an administrator terminal, it can also display the validity of the response information evaluated by the validity determination unit 161. That is, when the evaluation unit 160 displays the user's learning results evaluated by the learning evaluation unit 163, it can present whether the evaluation of the learning results is valid or invalid, or the probability of its validity or invalidity, thereby facilitating user learning management. Alternatively, if the validity determination unit 161 determines that the response information is invalid, the evaluation unit 160 can increase its operation speed by not evaluating the user's learning results and only displaying information indicating invalidity.

The validity determination unit 161 can evaluate the validity of the response information through evaluation criteria such as a conversation response speed evaluation criterion, a speech rate evaluation criterion, and a conversation repetition evaluation criterion. The conversation response speed evaluation criterion refers to an evaluation criterion for assessing whether the user responded at an appropriate time after the other party's speech ended. Not only when responding to the other party's speech after a long time has passed since it ended, but also when responding to the other party's speech before it has ended or immediately after it has ended, it may be a completely different conversation unrelated to the answer to the other party's speech. Therefore, when options are presented to the user terminal 10, if the user selects the position where an option will be presented even before it is displayed, or selects an option after a preset time has passed since it was displayed, the option information including the user's option selection can be evaluated as invalid. This can also be applied when the user terminal 10 provides an environment where the user can actually utter, allowing the user's utterance to be recorded to generate utterance information, and evaluating validity based on that utterance information. That is, even if the user's utterance began before or immediately after the other party's speech ended in each content, or began after a preset time had passed since the other party's speech ended, the user's utterance information can be evaluated as invalid.

In this case, when the validity determination unit 161 evaluates the validity of the utterance information based on the conversation response speed evaluation criterion, it can also apply a conversation repetition evaluation criterion. The conversation repetition evaluation criterion is an evaluation criterion for assessing whether the user repeats the same words or phrases when uttering. For example, if the user's utterance includes echolalia, the validity determination unit 161 determines that the user responded at the time the echolalia ended. Echolalia refers to the act of repeating or imitating the other person's words directly or similarly without being able to answer them. While echolalia in infancy (around 1 to 3 years old) is a normal phenomenon, its persistence after infancy is a characteristic symptom of patients with developmental disorders (autism spectrum disorder, language disorder, intellectual disability) and hinders smooth communication. In severe developmental disorders, echolalia may persist even into adulthood. Echolalia is divided into immediate echolalia, which appears immediately after hearing the other person's words, and delayed echolalia, which appears after some time. In this case, the types of echolalia may include, for example, types such as "A is A" or "A is A+a".

For example, if the other party's question is "What are you doing today?", and the user's utterance in response to that question includes parts that directly or similarly repeat the other party's question, such as "What are you doing today? What is today? What about today? Today I eat strawberries," the validity determination unit 161 judges only the subsequent utterance as the user's utterance, excluding the repeated part. Therefore, if the user's utterance includes echolalia, and the time interval from the end of the other party's speech to the end of the echolalia is longer than a preset time interval, the validity determination unit 161 can determine that the user's response information is invalid.

When evaluating the validity of utterance information, the validity determination unit 161 can evaluate its validity according to a speech rate evaluation criterion. The speech rate evaluation criterion is for evaluating the speed of speech during a conversation. Speech rate is an important skill for effectively conveying speech to the other party. If it is excessively fast or slow, it may be difficult for the other party to grasp the speaker's words or to pay attention to them, making conversation maintenance difficult, and it may also be a completely different conversation unrelated to the answer to the other party's speech. Therefore, if the user's speech rate is faster or slower than a preset rate, the validity determination unit 161 can determine that the user's response information is invalid.

The validity determination unit 161 can recognize the user's emotion from the user's utterance information. The validity determination unit 161 compares the recognized emotion during the user's utterance with the emotion included in the exemplary response information of the speaker performing the user's role within each content. If the emotions are the same, the validity determination unit 161 determines that the user's response information is valid; if they are different, it may determine that the response information is invalid. Meanwhile, for this purpose, the utterance information of the speaker performing the user's role within the content, i.e., the exemplary response information, includes not only the voice and text corresponding to the exemplary response but also information about the emotion corresponding to that exemplary response. In this case, the emotion information may be, for example, any one of neutral, joy, sadness, anger, disgust, surprise, or fear. Furthermore, the validity determination unit 161, by analyzing the user's utterance information, can recognize the user's emotion during utterance as any one of the aforementioned neutral, joy, sadness, anger, disgust, surprise, or fear.

The validity determination unit 161 analyzes the voice signal within the user's utterance information to recognize the user's emotional state. Voice-based emotion recognition technology is one of the voice processing application methods that can recognize the speaker's voice and identify the speaker's emotional or physical state from the recognized voice. The method of recognizing user's emotion from voice generally uses a Feature Extraction method, and the accuracy of emotion recognition from voice varies depending on the extracted features. Currently commonly used feature extraction methods can recognize emotional states from voice using speech Pitch, Speech Rate, Intensity, Mel-Frequency Cepstral Coefficients (MFCC), and Linear Prediction Cepstral Coefficients (LPCC), among others.

As an embodiment, the validity determination unit can divide an input data set corresponding to a voice signal into frames according to the characteristics of pre-designated emotions. For example, the validity determination unit can divide an input data set corresponding to voice signals of various different users collected in advance into frames corresponding to the user's emotions. For example, the validity determination unit can extract features corresponding to pre-designated valid sounds from an input data set corresponding to a voice signal based on Mel Frequency Cepstral Coefficient (MFCC).

MFCC is an algorithm invented by Davis and Mermelstein in the 1980s that extracts valid features of real sounds into a two-dimensional array. Instead of processing the entire sound, it divides the sound into short time segments, analyzes the spectrum of these segments, and converts the features into numerical data. MFCC data is designed not to distinguish differences in pitch, and is used in fields such as speech recognition, music genre analysis, and emotion recognition. That is, the validity determination unit does not extract features from the entire input data set, but divides it into specific intervals, i.e., certain frames, and extracts features through spectrum analysis for each frame. For instance, since voice signals continuously change in the time domain, to extract features from changing sounds, it can be assumed that the voice signal does not change much within a pre-designated short time. That is, it can be interpreted that there is practically no change in the voice signal within the error range. Then, the validity determination unit can calculate the power spectrum (i.e., frequency) for each frame. Here, the power spectrum calculated for each frame can be extracted as a feature, i.e., a feature vector. Thus, when neural frequency is calculated, it is possible to know how much energy exists in each segment. The validity determination unit divides the user's utterance information and exemplary response information into segments at the same ratio and compares the energy of each segment to recognize the user's emotion during utterance as any one of neutral, joy, sadness, anger, disgust, surprise, or fear.

The learning evaluation unit 163 can evaluate the user's learning results. The learning evaluation unit 163 can convert the voice signal included in the user's utterance information into text through a known method, and then compare it with the text included in the exemplary response information through similarity evaluation to determine how similar the user's utterance is to the exemplary response. In this case, the learning evaluation unit 163 can evaluate the user's learning results according to a keyword evaluation criterion. The keyword evaluation criterion is for identifying whether keywords are included in the user's utterance and evaluating them.

Meanwhile, in the case of individuals with autism spectrum disorder, there may be errors in their pronunciation (i.e., mispronunciation) when uttering, compared to neurotypical individuals. That is, accurate pronunciation (i.e., correct pronunciation) like that of neurotypical individuals is not easy for individuals with autism spectrum disorder. Pronunciation errors are a common phenomenon in toddlers who are unskilled at pronunciation. In infancy, difficult phonemes cannot be pronounced, so they are omitted or changed to easier phonemes. If pronunciation errors persist even after 7 years of age, it corresponds to a type of speech sound disorder, specifically articulation and phonological disorder. Articulation and phonological disorder is the most common type of speech disorder, and it often persists into adulthood.

The learning evaluation unit 163 analyzes the voice signal within the user's utterance information, and if a mispronounced word is included in the voice signal, it can replace that mispronounced word with a correctly pronounced word to evaluate the user's learning. For example, in the case of mispronunciation, examples include substitution, distortion, omission (deletion), addition, contraction, inversion, and repetition. In the case of substitution, it means replacing a standard sound with another standard sound, and in the case of distortion, it means replacing a standard sound with a non-standard sound (an inaccurate pronunciation that cannot be represented in Hangul). Examples of substitution include replacing a standard sound with another standard sound, such as 'sagwa -> dagwa', 'gabang -> gabam', 'eomma -> eommya'. An example of distortion includes replacing a standard sound with a non-standard sound, such as 'sagwa -> thagwa'. Omission (deletion) refers to the omission of phonemes, and examples include 'sagwa -> agwa', 'pungseon -> puseon', and so on. Addition refers to the addition of unnecessary phonemes or syllables, and examples include 'ottugi -> kottugi', 'namu -> nammu', 'haetda -> haesseuda', 'gawi -> gawiya', and so on. Contraction refers to the omission of syllables, and examples include 'gaeguri -> gaeri', 'elevator -> ebiteo', and so on. Inversion refers to changing the position of phonemes or syllables, and examples include 'satang -> tasang', 'jangnangam -> jannanggam', 'naengjanggo -> naenggojang', and so on. Repetition refers to repeating a specific syllable of a word, and examples include 'baji -> baba', 'jangnangam -> gamnanggam', and so on.

When the response information is utterance information and includes a mispronounced word as exemplified above, the learning evaluation unit 163 replaces that mispronounced word with a correctly pronounced word. For example, if 'gabam' is included in the utterance information, the mispronounced 'gabam' is replaced with the correctly pronounced 'gabang'. Or, if 'eommya' is included in the utterance information, the mispronounced 'eommya' is replaced with the correctly pronounced 'eomma'. Meanwhile, such replacement of mispronounced words with correctly pronounced words by the learning evaluation unit 163 is performed through a pre-learned database, and since the range of mispronunciations varies widely for each user, it may be difficult to replace all mispronunciations with correct pronunciations. Therefore, the digital therapeutic server 100 can receive and analyze pronunciation information from each user, and learn the user's mispronounced words and corresponding correctly pronounced words through the voice signals included in the pronunciation information. Therefore, the digital therapeutic system 1000 according to the present invention can provide customized replacement of mispronounced words for each user.

In detail, the digital therapeutic server 100 according to the present invention may further include a pronunciation unit 111 that receives pronunciation information from a user, analyzes it, learns from it, and stores the learning results in a database. The pronunciation unit 111 can transmit first data including text information that the user should respond to, to the user terminal 10. The user terminal 10 can display the text information included in the received first data, then record the user's utterance of that text information to generate second data including pronunciation information. The user terminal 10 transmits the generated second data to the pronunciation unit 111, and the pronunciation unit 111 can analyze, learn, and store the received second data. In this case, the text information that the user should respond to may refer to the textified exemplary responses of the speaker acting as the user's role within the skill reinforcement content and virtual field content, respectively, provided by the application unit 140 and the virtual field unit 150 to the user terminal 10. That is, before learning skills through basic content, skill reinforcement content, and virtual field content, the user receives and pronounces the text corresponding to the exemplary responses. At this time, the user may pronounce the text corresponding to all exemplary responses, or may receive and pronounce only a portion of the text from the exemplary responses.

The pronunciation unit 111 can analyze the second data including pronunciation information based on the text of only a portion of the exemplary responses, extract the user's mispronunciations, learn the correct pronunciations corresponding to those mispronunciations, and store the extracted and learned mispronunciations and their corresponding correct pronunciations in a database by linking them. Meanwhile, as an embodiment, the pronunciation unit 111 can transmit the pronunciation information received from the user terminal 10 and mispronounced words extracted from that pronunciation information to a guardian terminal (not shown). The guardian terminal can refer to the voice signal included in the received pronunciation information to determine exactly which correctly pronounced word the mispronounced word corresponds to, and transmit this determination to the pronunciation unit 111. Therefore, mispronounced words and their exact corresponding correctly pronounced words can be clearly linked. It would be desirable to request and link the correctly pronounced words corresponding to mispronounced words via the guardian terminal when the mispronounced words cannot be clearly determined within the system.

As described above, if the response information includes a mispronounced word, the learning evaluation unit 163 can evaluate the user's learning by replacing that mispronounced word with a correctly pronounced word. In this case, if the mispronounced word included in the response information matches a mispronounced word extracted from the pronunciation information received from the user terminal 10, the learning evaluation unit 163 replaces the mispronounced word included in the response information with the correctly pronounced word linked to the mispronounced word extracted from the pronunciation information to evaluate the user's learning. Thus, customized user evaluation is possible.

The second data may further include user's meta information along with pronunciation information. In this case, the meta information includes the user's age, gender, and disability level. Therefore, the pronunciation information can also be used when replacing mispronounced words of other users with similar age groups, the same gender, and similar disability levels with correctly pronounced words. Meanwhile, the pronunciation information is used not only for replacing mispronounced words with correctly pronounced words but also in evaluating the user's learning. Pronunciation information is generated when the user pronounces the text that the user should utter in the second skill reinforcement content and the second virtual field content. Subsequently, after the user acquires skills through the basic content, skill reinforcement content, and virtual field content, the user utters appropriate responses through the second skill reinforcement content and the second virtual field content, and the learning evaluation unit 163 evaluates those utterances.

As described above, the learning evaluation unit 163 can convert the voice signal included in the user's utterance information into text through a known method, and then compare it with the text included in the exemplary response information through similarity evaluation to determine how similar the user's utterance is to the exemplary response. In this case, the learning evaluation unit 163 can compare the user's utterances recorded through the second skill reinforcement content and the second virtual field content with the pronunciation information previously generated through pronunciation, using similarity evaluation, to determine how similar the user's utterances are to the previously pronounced pronunciation information, thereby making the evaluation of the learning results more accurate. Alternatively, when evaluating portions not pronounced by that user, it can evaluate based on pronunciation information of other users with similar age groups, the same gender, and similar disability levels stored in a database, by referring to meta information.

Hereinafter, other configurations of the digital therapeutic server 100 according to the present invention, excluding the pronunciation unit 111 and the evaluation unit 160, will be described in more detail.

The collection unit 110 can collect background information of a user with Autism Spectrum Disorder (ASD) through the user terminal 10 to ascertain the user's current knowledge. For example, the collection unit 110 collects the user's age, disability level, gender, and so on. Furthermore, the collection unit 110 may include the aforementioned pronunciation unit 111.

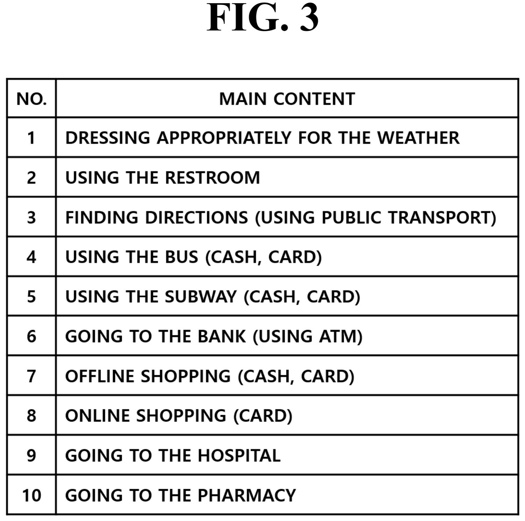

FIG. 3 is a diagram illustrating an example of a topic presented by the presenting unit 120 to the user terminal 10.

The presenting unit 120 presents at least one topic to the user terminal 10. The topics presented by the presenting unit 120 may be configured to allow practice of communication processes with people encountered in daily life or social situations, considering the social interaction and communication deficits of individuals with autism spectrum disorder. This corresponds to the objective of the present invention, which is to provide personalized smart healthcare services to individuals with autism spectrum disorder aged 10 to 18 who require intensive development of peer social situation recognition and interaction, rather than infants. As the topics presented by the presenting unit 120 are applied to situations that may occur in daily life, the basic content provided by the learning unit 130, which will be described later, also includes content about communication processes with people encountered in daily life or social situations, thereby comprehensively promoting planning and organization, cognitive flexibility, and social interaction in the process of individuals with autism spectrum disorder adapting to daily life.

As described above, the topics presented by the presenting unit 120 to the user terminal 10 may be topics that may occur in daily life, and for example, as shown in FIG. 3, may include ① dressing according to the weather, ② using the restroom, ③ finding directions (using public transport), ④ using the bus (cash, card), ⑤ using the subway (cash, card), ⑥ going to the bank (using ATM), ⑦ offline shopping (cash, card), ⑧ online shopping (card), ⑨ going to the hospital, and ⑩ going to the pharmacy.

When the presenting unit 120 presents the aforementioned topics to the user terminal 10, the user terminal 10 may select one of the topics presented by the presenting unit 120. Meanwhile, in one embodiment, a guardian of an individual with autism spectrum disorder, for example, a parent, may select one of the aforementioned topics. Although not shown in the drawings, if a guardian intervenes in the service provided by the present invention, the guardian may access the digital therapeutic server 100 through a guardian terminal (not shown).

When the user terminal 10 selects one of the aforementioned topics, the presenting unit 120 may receive information about that selection and transmit the information to the learning unit 130.

The learning unit 130 may provide a plurality of detailed tasks according to the topic to the user terminal 10.

In one embodiment, when the topic of 'dressing according to the weather' is selected, the learning unit 130 presents detailed tasks such as ① checking the weather, ② checking the temperature, ③ determining the general category of clothes, ④ choosing appropriate clothes from those I possess, and ⑤ checking for inappropriate clothes.

Alternatively, in one embodiment, when the topic of 'using the bus (card)' is selected, the learning unit 130 presents detailed tasks such as ① searching for the departure point and destination via Naver Map or Kakao Map application, ② checking the travel time, ③ preparing to depart 20 minutes earlier than the travel time, ④ checking the bus number to take, ⑤ checking the bus stop, ⑥ waiting for the bus at the bus stop, ⑦ holding the transportation card in hand beforehand, ⑧ tapping the transportation card after boarding when the bus arrives (e.g., a scene of dragging and tagging the transportation card), ⑨ sitting down, ⑩ checking the stop to alight, ⑪ listening carefully to the bus announcement and pressing the bell, and ⑫ tapping the card and alighting.

Alternatively, in one embodiment, when the topic of 'using the subway (card)' is selected, the learning unit 130 presents detailed tasks such as ① searching for the departure point and destination via Naver Map or Kakao Map application, ② checking the number of subway transfers, ③ checking the subway station, ④ checking the travel time, ⑤ preparing to depart 20 minutes earlier than the travel time, ⑥ checking the name of the alighting station, ⑦ listening carefully to the subway announcement and preparing to alight, ⑧ checking the map after alighting to see which exit to take, ⑨ exiting through the ticket gate after alighting when the announcement is made, by looking at the exit guide board, ⑩ tapping the transportation card when exiting, and ⑪ finding the exit by looking at the exit guide board.

Alternatively, in one embodiment, when the topic of 'going to the bank (using ATM - withdrawal)' is selected, the learning unit 130 presents detailed tasks such as ① checking the bank mark on the check card (e.g., bank names appear by bank mark), ② searching for the bank name on Naver Map or Kakao Map, ③ finding the nearest bank, ④ opening the door and entering where the ATM is located, ⑤ clicking the deposit/withdrawal button, ⑥ clicking "Card", ⑦ inserting the card into the blinking area in the direction of the arrow, ⑧ selecting the amount, ⑨ if the amount is not available, selecting 'Other' and then entering the number and pressing the confirm button, ⑩ confirming the amount and pressing confirm, ⑪ entering the 4-digit card PIN, ⑫ printing the statement, and ⑬ receiving the card and statement.

Alternatively, in one embodiment, when the topic of 'online shopping (card - app card installation guide)' is selected, the learning unit 130 presents detailed tasks such as ① searching for "mask" on Naver, ② clicking the shopping tab, ③ viewing desired color and size and clicking, ④ selecting color and size and confirming quantity to purchase, ⑤ clicking 'Purchase', ⑥ searching and entering home address for delivery address, ⑦ entering mobile phone number, ⑧ entering delivery message, ⑨ clicking 'General Payment', ⑩ selecting credit card, ⑪ clicking the company name of the card I possess, ⑫ clicking "Pay", ⑬ clicking "Pay with App Card", ⑭ pressing the QR code to pay, and ⑮ entering simple payment password.

Meanwhile, each task presented by the learning unit 130 may consist of a single scene in the basic content.

The learning unit 130 may provide basic content according to the topic selected by the user and the plurality of tasks related to that topic to the user terminal 10. The basic content provided by the learning unit 130 to the user terminal 10 may, as described above, consist of videos, each containing scenes corresponding to the tasks according to the topic selected by the user. The basic content may, for example, consist of animations in which multiple speakers converse with each other. The video of the basic content may also include, in addition to the conversations of multiple speakers, separate configurations to explain each topic and task and to help the user understand the current situation.

Meanwhile, the learning unit 130 may adjust the difficulty level of the basic content according to the user's background information collected by the collection unit 110. For example, the learning unit 130 adjusts the difficulty level of the basic content according to the user's age and disability level. In this case, the difficulty level of the basic content is adjusted by controlling the number of speakers or the number of tasks.

As the number of speakers increases, the amount of conversation exchanged also increases, and a higher level of conversational ability is required. For example, if there are two speakers, conversations can be exchanged alternately and gaze can be focused only on the other person, but if there are four speakers, one must remember the words of the three other people and respond according to the flow of the conversation, and conversation timing and gaze control become more complex.

Also, if the number of tasks increases, the difficulty level decreases, and if the number of tasks decreases, the difficulty level increases. For example, in the case of the task 'finding the exit after taking the subway', if it is divided into detailed tasks such as 'confirming the alighting station > listening carefully to the announcement > checking the exit on the map', the difficulty level for each task decreases.

Meanwhile, this adjustment of difficulty level is not only applied to the basic content of the learning unit 130 but may also be applied to the skill enhancement content of the application unit 140 and/or the virtual reality content of the virtual reality unit 150.

The application unit 140 may provide skill enhancement content that allows the application and utilization of skills corresponding to the basic content to the user terminal 10. The skill enhancement content may, as described above, be centered on a method where multiple speakers converse with each other to focus solely on conversation training based on the conversation content included in the basic content. In this case, one of the speakers may play the role of the user. When the speaker playing the user's role utters, the animation within the skill enhancement content is partially modified to indicate that speaker, to allow focus on that utterance. Regarding the display of the speaker, the application unit 140 may assign feature points to the user's face area captured by the camera of the user terminal 10, calculate and data-convert the change amount of the assigned feature points using an interpolation method, thereby implementing a real-time changing 2D or 3D model's face and/or facial expression animation, which will be described later.

When providing skill enhancement content to the user terminal 10, the application unit 140 may mute the utterance of the speaker who played the user's role in the skill enhancement content before providing it. That is, the application unit 140 may provide general skill enhancement content (e.g., 'first skill enhancement content') as described above to the user terminal 10 for a preset number of times determined according to the user's age and disability level, and then provide skill enhancement content (e.g., 'second skill enhancement content') where only the utterance of the speaker who played the user's role is muted, to the user terminal 10. The application unit 140 presents a video with the dialogue of the character representing the user omitted during video playback through the second skill enhancement content, and allows the user to learn linguistic conversational expressions by completing the omitted dialogue.

When the dialogue of the character representing the user is omitted in the second skill enhancement content, the application unit 140 may display a plurality of options on the user terminal 10 for the user to select the appropriate utterance content of that character. The user may select the option containing the appropriate utterance from the displayed options.

Alternatively, when the dialogue of the character representing the user is omitted in the second skill enhancement content, the application unit 140 may provide an environment where the user can actually utter the appropriate dialogue content of that character. The user terminal 10 may record the user's utterance when the muted utterance of the speaker is provided during playback of the second skill enhancement content. The user terminal 10 transmits the option information selected by the user and/or the recorded user's utterance to the application unit 140, and the application unit 140 transmits the received option information and/or the recorded user's utterance to the evaluation unit 160. The evaluation unit 160 may evaluate the option information selected by the user and/or the recorded user's utterance, as described above.

Furthermore, the virtual reality unit 150 may, as described above, provide virtual reality content, which is a reconfigured version of tasks that may be derived from topics and/or basic content or utterances from skill enhancement content, to the user terminal 10. The virtual reality content provides options for the user to select appropriate utterances in a virtual setting related to the topic presented by the presenting unit 120, or provides an environment where the user can actually utter, allowing the user to apply learned skills in a virtual setting. The virtual reality content is intended to teach and train individuals with autism spectrum disorder how to appropriately cope with various changing situations, considering their characteristic difficulty in flexibly adapting and applying to various subsequent changes.

For example, if the topic selected by the user is 'dressing according to the weather', the detailed tasks are, as described above, ① checking the weather, ② checking the temperature, ③ determining the general category of clothes, ④ choosing appropriate clothes from those I possess, and ⑤ checking for inappropriate clothes. The basic content is a video including scenes for each detailed task, and the skill enhancement content is constructed by extracting only the utterance content from each basic content. In this case, the virtual reality content may include contents derived from the aforementioned topics and tasks, for example, guiding how to borrow an umbrella or buy an inexpensive umbrella when the weather forecast differs from the actual weather, or when it suddenly rains. Furthermore, the virtual reality content may be a reconfigured version of the utterance content of the skill enhancement content, which was constructed by extracting only the utterance content from each basic content. Reconfiguration in this context may mean changing the speaker from female to male, changing the age group of the speaker, or changing the number of speakers. Therefore, the virtual reality content presents various changing situations based on topics and detailed tasks, and allows for the education and learning of correct utterances accordingly.

The virtual reality content may, like the aforementioned skill enhancement content, include a speaker playing the role of the user, and may include first virtual reality content in which the utterance content is fully included, and second virtual reality content in which the utterance of the speaker playing the role of the user is muted during utterance.

That is, the first virtual reality content may include a video containing content derived from the basic content and/or a reconfigured video of the skill enhancement content. The first virtual reality content is structured as multiple speakers conversing with each other, and one of the speakers may play the role of the user.

The second virtual reality content may be provided to the user with the utterance of the speaker who played the user's role in the first virtual reality content muted.

The virtual reality unit 150 may provide the first virtual reality content to the user terminal 10 for a preset number of times determined according to the user's age and disability level, and then provide the second virtual reality content, in which only the utterance of the speaker who played the user's role is muted, to the user terminal 10. The virtual reality unit 150 may present a video with the utterance of the character representing the user omitted during video playback through the second virtual reality content, and allow the user to learn linguistic conversational expressions by applying the utterances learned from the basic content and skill enhancement content to complete the omitted dialogue.

In this case, the virtual reality unit 150 may receive option information and/or utterance information selected or recorded through the virtual reality content from the user terminal 10, and the virtual reality unit 150 transmits the received option information and/or recorded utterance information to the evaluation unit 160. The evaluation unit 160 may evaluate the option information selected by the user and/or the recorded user's utterance according to criteria described later.

The evaluation unit 160 evaluates the content learned by the user based on the correctness of the option information received by the application unit 140 and/or the virtual reality unit 150, and/or the recorded user's utterance. The evaluation unit 160 analyzes and evaluates the audio of the recorded user's utterance content, and then determines the learning training as successful if the user's conversation content meets the preset evaluation criteria, and determines it as learning training failure if the user's conversation content does not meet the evaluation criteria. The detailed description of the evaluation unit 160 is as described above.

Meanwhile, the services provided by the collection unit 110, the presenting unit 120, the learning unit 130, the application unit 140, the virtual reality unit 150, and the evaluation unit 160 to the user terminal 10 correspond respectively to the six stages of cognitive behavioral therapy: Assessment or psychological assessment, Reconceptualization, Skills acquisition, Skills consolidation and application training, Generalization and maintenance, and Post-treatment assessment follow-up.

Cognitive behavioral therapy is one of the psychotherapeutic techniques that approaches human thought, behavior, and emotions based on their interrelationships. Cognitive behavioral therapy is known to yield results such as cognitive improvement, behavior modification, and emotional regulation through repetitive learning, and is an evidence-based therapy currently utilized for various mental disorders such as ADHD, mood disorders, and addictions.

Cognitive Behavioral Therapy (CBT) is one of the psychotherapeutic techniques that approaches human thought, behavior, and emotions based on their interrelationships, through six stages: Assessment or psychological assessment, Reconceptualization, Skills acquisition, Skills consolidation and application training, Generalization and maintenance, and Post-treatment assessment follow-up. Cognitive behavioral therapy is known to yield results such as cognitive improvement, behavior modification, and emotional regulation through repetitive learning, and is an evidence-based therapy currently utilized for various mental disorders such as ADHD, mood disorders, and addictions.

For example, the collection unit 110 identifies the user's current knowledge, corresponding to the 'Assessment or psychological assessment' stage of cognitive behavioral therapy. The collection unit 110 can identify the user's current knowledge and collect information such as disability level. In addition, the collection unit 110 can also collect the user's age, gender, and so on.

The presenting unit 120 may present topics to be learned, corresponding to the 'Reconceptualization' stage of cognitive behavioral therapy. Meanwhile, reconceptualization refers to the process of re-evaluating and restructuring how a user understands and deals with current problematic situations. Therefore, the topics presented by the presenting unit 120 can be selected based on situations that may occur in the user's daily life.

The learning unit 130 may, corresponding to the 'Skills acquisition' stage of cognitive behavioral therapy, present detailed tasks when the user selects a topic, and provide basic content containing the details of those tasks to the user. In this case, the basic content enables the acquisition of skills in detailed tasks through repetitive practice.

The application unit 140 may, corresponding to the 'Skills consolidation and application training' stage of cognitive behavioral therapy, provide skill enhancement content consisting only of utterance content included in the basic content to the user. In this case, the skill enhancement content provides options for the user to select appropriate utterances, or provides an environment where the user can actually utter, allowing the application of learned skills to real situations.

The virtual reality unit 150 may, corresponding to the 'Generalization and maintenance' stage of cognitive behavioral therapy, provide virtual reality content that enables repetitive practice in a virtual setting for tasks that may be derived from basic content and skill enhancement content, or for situations that may additionally arise for each topic. The virtual reality content provides options for the user to select appropriate utterances in a virtual setting related to the topic presented by the presenting unit 120, or provides an environment where the user can actually utter, allowing the user to apply learned skills in a virtual setting.

The evaluation unit 160 may allow for the evaluation and repetition of the entire process, corresponding to the 'Post-treatment assessment follow-up' stage of cognitive behavioral therapy. For example, the evaluation by the evaluation unit 160 may evaluate the user's education and learning outcomes based on the options selected by the user and/or the content actually uttered by the user during the skill enhancement content of the application unit 140 and/or the virtual reality content of the virtual reality unit 150.

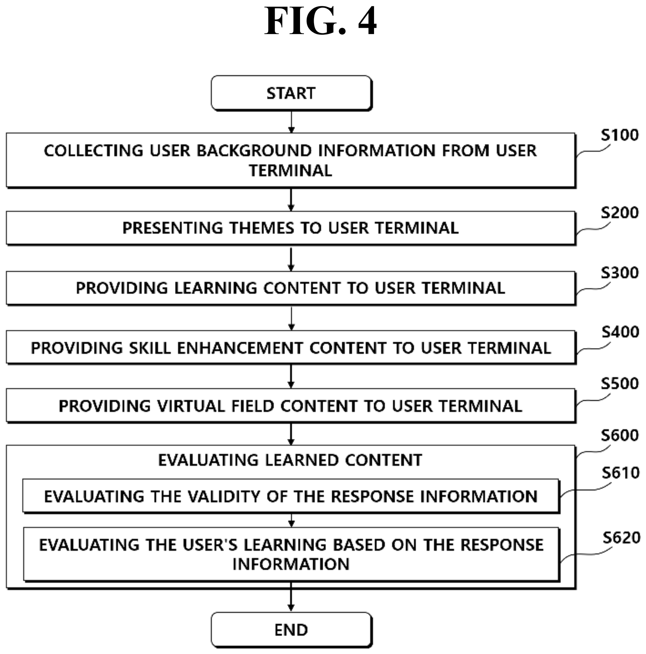

FIG. 4 is a flowchart of a Cognitive Behavioral Therapy-based digital treatment method according to the present invention.

The Cognitive Behavioral Therapy-based digital treatment method (hereinafter, referred to as 'method') according to the present invention, referring to FIG. 4, may include: a step (S100) where a collection unit 110 collects user's background information from a user terminal 10; a step (S200) where a presentation unit 120 presents at least one topic to the user terminal 10; a step (S300) where, if the user terminal 10 selects one of the topics, a learning unit 130 provides a plurality of tasks and basic content corresponding to the selected topic to the user terminal 10; a step (S400) where an application unit 140 provides skill enhancement content that can apply skills corresponding to the basic content to the user terminal 10; a step (S500) where a virtual field unit 150 provides virtual field content derived from the basic content or with content of the skill enhancement content reconstructed to the user terminal 10; and a step (S600) where an evaluation unit 160 evaluates the user's response information received from the user terminal 10 to evaluate the learning result of the user terminal 10.

In particular, step S600 may include a step (S610) where a validity determination unit 161 evaluates the validity of the response information, and a step (S620) where the user's learning is evaluated based on the response information. In this case, in step S610, the validity of the response information may be evaluated based on a conversation response speed evaluation standard that evaluates whether the user responded at an appropriate time after the other party's speech ended.

The details of each step are as described above. For example, step S100 may include a step (not shown) where a pronunciation reading unit 111 transmits first data containing text information to be uttered by the user to the user terminal 10, and a step (not shown) where the user terminal 10 generates second data containing pronunciation reading information generated by the user reading the text information included in the received first data and transmits it to the pronunciation reading unit 111.

Meanwhile, the method may further include a step (not shown) of providing a reward to the user after step S600.

The step of providing a reward to the user can involve giving a first reward to the user terminal 10 once the evaluation by the evaluation unit 160 is completed.

In this case, the step of providing a reward to the user can involve giving a first reward along with a second reward to the user terminal 10 when the user's conversation content is determined to be a successful learning training because it conforms to preset evaluation criteria by the evaluation unit 160.

To this end, the system according to the present invention may further include a reward unit (not shown).

The reward unit can provide a first reward to the user terminal 10 upon receiving evaluation result information from the evaluation unit 160. In this case, the first reward can be given regardless of the result when the user has completed learning a topic and the tasks associated with that topic. Therefore, the size of the first reward may be smaller than other rewards to be described later.

On the other hand, the second reward is given when the user achieves a result that meets the evaluation criteria for a topic and its associated tasks, resulting in successful learning training, and thus it may be a larger reward than the first reward. The reward unit that provides the second reward to the user transmits the results to the collection unit 110, the presentation unit 120, and/or the learning unit 130. If the user receives the second reward, the collection unit 110 collects and stores this as user's background information. If the user receives the second reward, the presentation unit 120 may mark the topic for which the second reward was received as successful learning training for a certain period, or may not present that topic to the user terminal 10 for a certain period. If the user selects the topic for which the second reward was received again, the learning unit 130 can increase the difficulty level of the basic content for that topic. Therefore, as described above, basic content with an increased number of speakers or a reduced number of tasks can be provided to the user terminal 10. Meanwhile, for individuals with Autism Spectrum Disorder, repeated learning of the same topic and tasks is important. If the user repeatedly learns basic content for a topic and tasks for which the difficulty level has been increased after receiving a second reward, and the evaluation unit 160 again evaluates it as successful learning training, the evaluation unit 160 provides a third reward to the user. Therefore, the reward unit not only encourages participation in learning by providing rewards for repeated learning, but also motivates voluntary repeated participation in topics and tasks learned within a certain period.

Meanwhile, the aforementioned first to third rewards may also be disbursed by a guardian terminal. For example, each reward may be applied as mileage or points that can be exchanged for physical rewards. Alternatively, each reward may take the form of a symbolic icon that can be directly exchanged for a physical reward. For example, such a symbolic icon may represent a physical item that the user likes, such as chocolate, strawberries, or snacks, and the guardian terminal can directly provide that reward to the user through the icon received by the user. This allows for the provision of tangible rewards to the user, thereby motivating learning participation and repeated participation.

In addition, for example, if each reward is mileage or points, the reward unit can categorize the topics learned by the user and determine topics with high similarity among them as similar topics.

The reward unit can separately calculate the reward amount based on the difficulty level and average time spent on similar topics. The calculated reward amount is determined as an estimated reward amount and can be displayed on the user terminal 10 along with the topic when the user selects a topic.

This is to provide a larger reward amount to the user when similar topics are repeatedly learned. It also has the characteristic of increasing learning efficiency by learning similar topics.

Meanwhile, as described above, the application unit 140 and the virtual field unit 150 can each provide skill enhancement content and virtual field content to the user terminal 10. In this case, the skill enhancement content and virtual field content include a speaker who performs the user's role. At this time, the application unit 140 and/or the virtual field unit 150 can recognize the user's face or expression and provide an animation to the user terminal 10 in which the user's current face or expression is synthesized in real time onto the face of the speaker performing the user's role.

The user terminal 10 photographs the user's face during learning through a camera and transmits the photographed face to the digital therapeutic system 1000. The application unit 140 and/or the virtual field unit 150 synthesize the received user's face or expression onto the face of the speaker performing the user's role within the animation.

Accordingly, the user can recognize the speaker with their current face or expression synthesized as the speaker performing their own role, and furthermore, this in itself can draw the user's gaze to that speaker, thereby improving learning efficiency. This not only provides fun in learning to the user, but also allows for real-time confirmation that the user is watching the skill enhancement content and/or virtual field content within the correct area.

The application unit 140 and the virtual field unit 150 can implement 2D or 3D model face and/or expression animations that change in real time by assigning feature points to the face region captured by the user terminal 10's camera and calculating and digitizing the change amount of the assigned feature points using interpolation.

The application unit 140 and the virtual field unit 150 can each search for the user's face region in an image captured by the user terminal 10's camera, assign feature points to the detected face region, digitize the change amount of the feature points when input, and apply the digitized change amount to a preset 2D or 3D model with feature points and control points corresponding to the change amount, thereby implementing a real-time changing 2D or 3D model's face and/or expression animation. The application unit 140 and the virtual field unit 150 each, when applying the digitized change amount, load a preset 2D or 3D model with feature points and control points, control and output the change amount of the feature points by the preset control points when the digitized change amount of the feature points is recognized, and if the change amount of the feature points is not transmitted, output the initialized face and/or expression of the loaded model. In this case, it is preferable that the digitized change amount is a vector function value with respect to time.

Hereinafter, a more specific description will be given of how the application unit 140 and the virtual field unit 150 implement real-time synthesis of the user's current face or expression onto the face of the speaker performing the user's role.

The application unit 140 and the virtual field unit 150 can each capture a face and/or expression through a camera to detect a face region, and assign feature points to the eyes, nose, mouth, eyebrows, and overall facial contour within the detected face region. Then, by digitizing the change amount of the feature points that change as the face and/or expression changes, and applying this to a preset 2D or 3D model with feature points and control points corresponding to the change amount of the feature points, they can implement a real-time changing 2D or 3D model face and/or expression animation.

When applying the digitized change amount of the feature points, a preset 2D or 3D model with feature points and control points that will change according to the change amount of the feature points is loaded, it is confirmed whether the digitized change amount of the feature points is received, and if received, the change amount of the feature points can be controlled and output by the preset control points. If the change amount of the feature points is not received, the initialized face and/or expression animation of the loaded model is output. Therefore, it can be recognized if the user is not watching the skill enhancement content and virtual field content or is watching outside a certain area.

Meanwhile, the change amount of feature points within the face region captured by the camera can be calculated through interpolation.

Interpolation is a technique for generating face expression animation by approximating intermediate steps from a face and/or expression represented by multiple feature points, and there are various known methods such such as shape interpolation that approximates geometric structures, and key frame interpolation that approximates images over time.

Hereinafter, one embodiment of the process to which interpolation is applied will be described in detail.

First, still images are extracted from real-time incoming video through a camera at regular time intervals, and feature points are assigned within the detected face region in the still images and temporarily stored in a first data memory. Then, after a certain time (e.g., 0.1 seconds to 1 second) has elapsed, still images are extracted from the incoming video, feature points are assigned within the detected face region and temporarily stored in a second data memory. After that, the feature points temporarily stored in the first data memory and the second data memory are compared, their change amount is calculated and digitized. At this time, the change amount is approximated as a vector function value with respect to time. Then, a preset 2D or 3D model with feature points is loaded and the digitized change amount of the feature points is applied to implement face expression animation.

Furthermore, the validity determination unit 161 can recognize the user's expression through the change amount of feature points within the face region captured by the camera, and can extract the user's emotion during utterance by analyzing the recognized user's expression. Therefore, the validity determination unit 161 can extract the user's emotion during utterance more accurately through the user's expression during utterance, along with the analysis of the aforementioned response information.

The user terminal 10 and guardian terminal according to the present invention are not limited to desktop PCs, laptop PCs, tablet PCs, smartphones, etc., equipped with input means such as a keyboard, mouse, touchpad, and touch screen, and a display screen, but may include any configuration capable of digital information processing that can connect to the digital treatment server 100 via a communication network, and install application programs that can input search information and selection information and display searched result information. The user terminal 10 according to the present invention is a component that connects to the digital treatment server 100 via a communication network to transmit and receive information, and for example, may include at least one of a smartphone, a tablet personal-computer, a mobile phone, a video phone, a desktop-personal computer, a laptop personal computer, a netbook computer, a personal digital assistant (PDA), a portable multimedia player (PMP), a wearable device (e.g., smart glasses, a head-mounted-device (HMD), etc.), a kiosk, or a smart watch.

The user terminal 10 and the digital treatment server 100 according to the present invention can communicate through a communication unit and a communication network provided in each of them. A communication network refers to a connected structure that enables information exchange between respective nodes such as terminals and servers. Examples of such a communication network include, but are not limited to, 3GPP (3rd Generation Partnership Project) network, LTE (Long Term Evolution) network, 5G network, WIMAX (World Interoperability for Microwave Access) network, Internet, LAN (Local Area Network), Wireless LAN (Wireless Local Area Network), WAN (Wide Area Network), PAN (Personal Area Network), Wi-Fi network, Bluetooth network, satellite broadcasting network, analog broadcasting network, DMB (Digital Multimedia Broadcasting) network, and the like. The communication unit provided in each of the user terminal 10 and the digital treatment server 100 may include electronic components provided for the communication network to perform wired/wireless data communication through the aforementioned communication network.

In this specification, 'unit' includes a unit realized by hardware, a unit realized by software, and a unit realized by using both. Also, one unit may be realized by using two or more hardware components, and two or more units may be realized by one hardware component.

The scope of protection of the present invention is not limited by the description and expressions of the embodiments explicitly described above. Furthermore, it is once again added that the scope of protection of the present invention cannot be limited by obvious changes or substitutions in the technical field to which the present invention belongs.

## Claims

1. A digital therapeutic system based on cognitive behavioral therapy, comprising:
a user terminal; and a digital therapeutic server configured to provide learning content based on cognitive behavioral therapy to the user terminal,
wherein the digital therapeutic server includes an evaluation unit configured to evaluate a user's learning result based on user response information received from the user terminal, wherein the user response information is selection option information or utterance information received from the user terminal, and
wherein the evaluation unit includes: a validity determination unit configured to evaluate the validity of the response information; and a learning evaluation unit configured to evaluate the user's learning based on the response information.

2. The digital therapeutic system of claim 1, wherein the validity determination unit is configured to evaluate the validity of the response information based on a conversation response speed evaluation criterion that evaluates whether the user responded at an appropriate time after an interlocutor's speech ended.

3. The digital therapeutic system of claim 2, wherein when evaluating the validity of the response information based on the conversation response speed evaluation criterion, the validity determination unit is configured to determine that the user responded at a time when echolalia included in the response information ended, in a case where the response information includes the echolalia.

4. The digital therapeutic system of claim 1, wherein when evaluating the validity of the response information, the validity determination unit is configured to extract emotion from the user's utterance by analyzing the response information, and evaluate the validity of the response information by comparing the extracted emotion with an emotion included in model response information.

5. The digital therapeutic system of claim 1, wherein when evaluating the user's learning, the learning evaluation unit is configured to evaluate a similarity between the user's response information and a model response.

6. The digital therapeutic system of claim 5, wherein in a case where the response information includes a mispronounced word, the learning evaluation unit is configured to replace the mispronounced word with a correctly pronounced word to evaluate the user's learning.

7. The digital therapeutic system of claim 6, wherein the digital therapeutic server further includes a pronunciation unit configured to receive pronunciation information from the user,
wherein the pronunciation unit is configured to transmit first data including text information to be uttered by the user to the user terminal, and
wherein the user terminal is configured to generate second data including pronunciation information generated by having the user pronounce the text information included in the received first data, and transmit the second data to the pronunciation unit.

8. The digital therapeutic system of claim 7, wherein the pronunciation unit is configured to analyze the pronunciation information to extract a mispronounced word of the user, and store the mispronounced word by matching it with a correctly pronounced word corresponding to the mispronounced word, and
wherein the learning evaluation unit is configured to evaluate the user's learning by replacing the mispronounced word included in the response information with the correctly pronounced word based on the mispronounced word extracted from the pronunciation information.

9. The digital therapeutic system of claim 8, wherein the second data further includes user meta information,
wherein the meta information includes user's age, gender, and disability level information.

10. The digital therapeutic system of claim 8, wherein when evaluating the user's learning, the learning evaluation unit is configured to evaluate a similarity between the user's response information and the pronunciation information.
